# EUROPEAN PATENT SPECIFICATION

(11) **EP 3 942 950 B1**
(45) Date of publication and mention of the grant of the patent: **11.11.2026**
(21) Application number: 21194151.3
(22) Date of filing: 27.07.2018
(51) Int. Cl.: A24F 40/46, A24F 40/70, A61M 15/06, A24F 40/20

(54) **HEATER ASSEMBLY WITH CAVITY**
HEIZUNGSANORDNUNG MIT HOHLRAUM
ENSEMBLE DE CHAUFFAGE AVEC CAVITÉ

(30) Priority: 28.07.2017 EP 17183836
(43) Date of publication of application: 26.01.2022
(62) Document number(s) of the earlier application(s) in accordance with Art. 76 EPC: 18745949.0 / 3 657 967
(73) Proprietor: Philip Morris Products S.A., 2000 Neuchâtel (CH)
(72) Inventor: AW, Sze Chiek, 543298 Sengkang (SG); FRINGELI, Jean-Luc, 307465 Newton (SG); KIAN, Yeo Chow, 807159 Yio Chu Kang (SG)
(74) Representative: Reddie & Grose LLP

(56) References cited:
- WO-A1-2015/197627
- WO-A1-2016/124552
- US-A1- 2014 261 408

## Description

The present invention relates to a heater assembly for an aerosol-generating device, the heater assembly having a cavity. The present invention also relates to an aerosol-generating device comprising the heater assembly, an aerosol-generating system comprising the aerosol-generating device, a method of generating an aerosol, and a method of assembling a heater assembly.

One type of aerosol-generating system is an electrically operated smoking system. Known handheld electrically operated smoking systems typically comprise an aerosol-generating device comprising a battery, control electronics and an electric heater for heating a smoking article designed specifically for use with the aerosol-generating device. In some examples, the smoking article comprises a plug of an aerosol-forming substrate, such as a tobacco plug, and the heater contained within the aerosol-generating device is inserted into the aerosol-forming substrate when the smoking article is inserted into the aerosol-generating device.

WO 2015/197627 A1 describes an aerosol-generating system comprising an aerosol-generating article comprising a nicotine source and a lactic acid source. The aerosol-generating system also comprises an aerosol-generating device comprising a cavity for receiving the aerosol-generating article and a single heater for heating the nicotine source and the lactic acid source. The single heater is an elongate electric heating element in the form of a heater blade positioned centrally within the cavity.

WO 2016/124552 A1 describes an electrically-heated aerosol-generating device comprising a heating assembly. The heating assembly comprises a heater and a heater mount. The heater mount provides structural support to the heater and allows the heater to be located within the aerosol-generating device. The heater mount is formed from a mouldable material that is moulded around a portion of the heater and extends through a though-hole to couple the heater to the heater mount. The use of such a through-hole increases the anchoring of the heater to the heater mount.

Aerosol-generating articles for electrically operated smoking systems typically exhibit a high liquid content to facilitate generation of an aerosol when heated. During use, liquid from the aerosol-generating article may collect within the aerosol-generating device, either through direct transfer from the aerosol-generating article, or as a result of the condensation of aerosol within the aerosol-generating device. The liquid that collects within the aerosol-generating device during use may leak into the interior of the aerosol-generating device and result in the deterioration or failure of electrical components within the aerosol-generating device.

It would be desirable to provide a heater assembly for an aerosol-generating device that reduces or eliminates the potential for the leakage of liquid into the aerosol-generating device during use.

According to a first aspect of the present invention there is provided a heater assembly for an aerosol-generating device, the heater assembly according to claim 1.

Advantageously, providing a heater assembly comprising a bushing facilitates the installation of the elongate electrical heater within a housing of an aerosol-generating device. That is, the bushing can be sized and shaped to fit within a housing of an aerosol-generating device.

Advantageously, the aperture through which a portion of the elongate electrical heater extends can facilitate correct positioning of the elongate electrical heater with respect to the bushing. Advantageously, this facilitates correct positioning and orientation of the elongate electrical heater when the heater assembly is installed within a housing of an aerosol-generating device.

Advantageously, the cavity into which a portion of the elongate electrical heater extends facilitates a seal between the elongate electrical heater and the bushing. That is, the cavity can be filled with a sealing material to reduce or eliminate the risk of liquid leakage through the heater assembly between the elongate electrical heater and the bushing.

Advantageously, the first flange may engage a seal between the bushing and a housing of an aerosol-generating device to reduce or prevent liquid leakage between the bushing and the housing of the aerosol-generating device.

Preferably, the heater assembly further comprises a potting compound positioned within the cavity. Advantageously, the potting compound provides a seal between the elongate electrical heater and the bushing. Advantageously, the potting compound can maintain the seal at elevated temperatures to which the elongate electrical heater is typically heated during use.

Preferably, the potting compound substantially fills the cavity. Advantageously, substantially filling the cavity with the potting compound may maximise the strength of the seal formed by the potting compound.

Preferably, the potting compound comprises at least one of a thermoset polymer, an epoxy resin, silicone, and combinations thereof. Advantageously, these potting compounds remain stable at elevated temperatures to which the elongate electrical heater is typically heated during use. Advantageously, these potting compounds can be deposited within the cavity in a liquid form and then set, for example by drying or curing. Advantageously, this facilitates ingress of the potting compound into spaces between the elongate electrical heater and the bushing to optimise the seal provided by the potting compound.

Preferably, the potting compound comprises at least one silicone. Advantageously, silicones retain a degree of elasticity after curing, which facilitates a robust seal between the bushing and the elongate electrical heater.

The potting compound may comprise a neutral cure silicone. Neutral cure silicones may exhibit increased elasticity after curing when compared to other silicones, such as acetoxy silicones. Advantageously, an increased elasticity may improve the reliability of the seal provided by the potting compound.

The potting compound may comprise an acetoxy silicone. Acetoxy silicones typically exhibit a reduced curing time when compared to neutral cure silicones. Advantageously, a reduced curing time facilitates manufacture of the heater assembly.

The portion of the elongate electrical heater extending through the aperture may be received within the aperture by an interference fit. That is, the interior cross-sectional dimensions of the aperture may be substantially the same as the exterior cross-sectional dimensions of the portion of the elongate electrical heater extending through the aperture.

Advantageously, an interference fit may secure the elongate electrical heater to the bushing. Advantageously, this may facilitate the use of an elastic potting compound in embodiments in which the heater assembly comprises a potting compound. That is, the interference fit may eliminate the need for the potting compound to secure the elongate electrical heater to the bushing.

Advantageously, an interference fit may prevent relative movement between the elongate electrical heater and the bushing.

The bushing comprises a first flange extending around either the first end of the bushing or the second end of the bushing. Preferably, the first flange extends around the first end of the bushing. Advantageously, this may facilitate engagement between the first flange and a seal when the heater assembly is inserted into a device cavity defined by a housing of an aerosol-generating device. That is, the second end of the heater assembly may be inserted into the device cavity so that a face of the first flange facing away from the first end of the bushing engages a seal.

Preferably, the first flange extends around the first end of the bushing and the bushing further comprises a second flange extending around the second end of the bushing, wherein an annular groove is formed between the first flange and the second flange. Advantageously, the annular groove may receive a gasket to facilitate sealing between the exterior of the bushing and an interior of a housing of an aerosol-generating device.

Preferably, the heater assembly comprises an annular gasket positioned within the annular groove. The annular gasket may be an O-ring. Preferably, the annular gasket is formed from a resilient material. Preferably, the annular gasket is formed from an elastomeric material. The annular gasket may comprise a rubber.

The bushing may be substantially cylindrical. Advantageously, a substantially cylindrical bushing may facilitate insertion of the heater assembly into a device cavity formed by a housing of an aerosol-generating device.

The bushing may comprise a first end face through which the aperture is formed, a second end face in which the cavity is formed, and a substantially cylindrical surface extending between the first end face and the second end face.

In embodiments in which the bushing comprises an annular groove, preferably the annular groove is formed in the substantially cylindrical surface. That is, first and second ends of the substantially cylindrical surface on either side of the annular groove form the first and second flanges respectively.

Preferably, the heater assembly further comprises at least one electrical contact positioned at the second end of the bushing, wherein the at least one electrical contact is electrically connected to the elongate electrical heater. Advantageously, the at least one electrical contact facilitates electrical connection of the elongate electrical heater to at least one of a power supply and control electronics of an aerosol-generating device.

Preferably, the at least one electrical contact comprises at least two electrical contacts.

Preferably, the bushing comprises at least one cut-out positioned on an edge of the bushing. Advantageously, the at least one cut-out may facilitate correct orientation of the heater assembly with respect to a housing of an aerosol-generating device during assembly of an aerosol-generating device comprising the heater assembly. That is, the at least one cut-out may engage at least one projection formed by a housing of an aerosol-generating device.

In embodiments in which the bushing comprises a first flange, the at least one cut-out may be provided on the first flange.

In embodiments in which the bushing comprises a first flange and a second flange, the at least one cut-out may be provided on the first flange, the second flange, or the first flange and the second flange. Preferably, the at least one cut-out comprises at least one cut-out provided on the second flange.

The bushing may be formed from any suitable material or combination of materials. Suitable materials include, but are not limited to, aluminium, polyether ether ketone (PEEK), polyimides, such as Kapton^{®}, polyethylene terephthalate (PET), polyethylene (PE), high-density polyethylene (HDPE), polypropylene (PP), polystyrene (PS), fluorinated ethylene propylene (FEP), polytetrafluoroethylene (PTFE), polyoxymethylene (POM), epoxy resins, polyurethane resins, vinyl resins, liquid crystal polymers (LCP) and modified LCPs, such as LCPs with graphite or glass fibres.

The bushing may be formed as a single part. The bushing may be formed from at least two parts secured together. The bushing may comprise a first part secured to a second part. The first part may be secured to the second part by at least one of an adhesive, a weld, and an interference fit.

A first portion of the aperture may be formed by the first part of the bushing and a second portion of the aperture may be formed by the second part of the bushing. A first portion of the cavity may be formed by the first part of the bushing and a second portion of the cavity may be formed by the second part of the bushing.

Advantageously, embodiments in which the bushing is formed from a first part secured to a second part may facilitate assembly of the heater assembly. For example, the elongate electrical heater may be positioned between the first part of the bushing and the second part of the bushing when the first part of the bushing is secured to the second part of the bushing. In embodiments in which the portion of the elongate electrical heater extending through the aperture is received within the aperture by an interference fit, this method of assembling the heater assembly may optimise the interference fit without damaging the elongate electrical heater. That is, the two-part assembly of the bushing may reduce or eliminate damage to the elongate electrical heater compared to embodiments in which the elongate electrical heater is inserted through the aperture formed in a single-part bushing.

Preferably, the elongate electrical heater comprises a heating portion extending from the first end of the bushing. Preferably, the heating portion is configured for insertion into an aerosol-generating article. The heating portion may comprise a tapered portion at an end of the heating portion distal from the first end of the bushing. The heating portion may be in the form of a pin. The heating portion may be in the form of a blade. The heating portion may be in the form of a cone.

The elongate electrical heater may be a resistive heater. The elongate electrical heater may comprise an electrically insulating substrate and a resistive heating element provided on the electrically insulating substrate.

Preferably, the electrically insulating substrate is operable at a working temperature of up to about 700 degrees Celsius, more preferably about 800 degrees Celsius. Additionally, or alternatively, the operating temperature of the resistive heating element during use may be about 250 degrees Celsius, more preferably about 300 degrees Celsius.

The electrically insulating substrate may be a ceramic material such as Zirconia or Alumina. Preferably, the electrically insulating substrate has a thermal conductivity of less than or equal to about 2 Watts per metre Kelvin.

The electrically insulating substrate may comprise a planar surface on which the resistive heating element is positioned and a tapered end configured to allow for insertion of the elongate electrical heater into an aerosol-generating article.

Suitable materials for forming the resistive heating element include but are not limited to: semiconductors such as doped ceramics, electrically "conductive" ceramics (such as, for example, molybdenum disilicide), carbon, graphite, metals, metal alloys and composite materials made of a ceramic material and a metallic material. Such composite materials may comprise doped or undoped ceramics. Examples of suitable doped ceramics include doped silicon carbides. Examples of suitable metals include titanium, zirconium, tantalum and metals from the platinum group. Examples of suitable metal alloys include stainless steel, nickel-, cobalt-, chromium-, aluminium- titanium- zirconium-, hafnium-, niobium-, molybdenum-, tantalum-, tungsten-, tin-, gallium-, manganese- and iron-containing alloys, and super-alloys based on nickel, iron, cobalt, stainless steel, Timetal^{®} and iron-manganese-aluminium based alloys.

In some embodiments, the resistive heating element comprises one or more stamped portions of electrically resistive material, such as stainless steel. Alternatively, the resistive heating element may comprise a heating wire or filament, for example a Ni-Cr (Nickel-Chromium), platinum, tungsten or alloy wire.

In embodiments in which the heater assembly comprises at least one electrical contact, preferably the resistive heating element is electrically connected to the at least one electrical contact.

According to a second aspect of the present invention there is provided an aerosol-generating device comprising a heater assembly according to the first aspect of the present invention, in accordance with any of the embodiments described herein. The aerosol-generating device and the heater assembly may comprise any of the optional or preferred features described with respect to the first aspect of the present invention.

The aerosol-generating device comprises a housing defining a device cavity for receiving an aerosol-generating article. The heater assembly is positioned within the housing at an upstream end of the device cavity, wherein the elongate electrical heater extends from the first end of the bushing into the device cavity. The aerosol-generating device also comprises a power supply and a controller for controlling a supply of electrical power from the power supply to the elongate electrical heater.

Preferably, the power supply and the control electronics are positioned upstream of the second end of the bushing. That is, preferably, the power supply and the control electronics are separated from the device cavity by the heater assembly.

In embodiments in which the heater assembly comprises a potting compound, advantageously the potting compound reduces or prevents the leakage of liquid from the device cavity through the heater assembly towards the control electronics and the power supply.

Preferably, the aerosol-generating device comprises a gasket positioned between the bushing and the housing. The gasket may form part of the heater assembly. The gasket may be positioned within an annular groove on the bushing, as described herein. Advantageously, the gasket reduces or prevents the leakage of liquid from the device cavity around the outside of the heater assembly towards the control electronics and the power supply.

The power supply may be a DC voltage source. In preferred embodiments, the power supply is a battery. For example, the power supply may be a nickel-metal hydride battery, a nickel cadmium battery, or a lithium based battery, for example a lithium-cobalt, a lithium-iron-phosphate or a lithium-polymer battery. The power supply may alternatively be another form of charge storage device such as a capacitor. The power supply may require recharging and may have a capacity that allows for the storage of enough energy for use of the aerosol-generating device with one or more aerosol-generating articles.

Preferably, the aerosol-generating device comprises at least one air inlet. Preferably, the at least one air inlet is in fluid communication with an upstream end of the device cavity.

The aerosol-generating device may comprise a sensor to detect air flow indicative of a consumer taking a puff. The air flow sensor may be an electro-mechanical device. The air flow sensor may be any of: a mechanical device, an optical device, an opto-mechanical device and a micro electro-mechanical systems (MEMS) based sensor. The aerosol-generating device may comprise a manually operable switch for a consumer to initiate a puff.

The aerosol-generating device may comprise a temperature sensor. The temperature sensor may detect the temperature of the elongate electrical heater or the temperature of an aerosol-generating article received within the device cavity. The temperature sensor may be a thermistor. The temperature sensor may comprise a circuit configured to measure the resistivity of the elongate electrical heater and derive a temperature of the elongate electrical heater by comparing the measured resistivity to a calibrated curve of resistivity against temperature.

Preferably, the aerosol-generating device comprises an indicator for indicating when the elongate electrical heater is activated. The indicator may comprise a light, activated when the elongate electrical heater is activated.

The aerosol-generating device may comprise at least one of an external plug or socket and at least one external electrical contact allowing the aerosol-generating device to be connected to another electrical device. For example, the aerosol-generating device may comprise a USB plug or a USB socket to allow connection of the aerosol-generating device to another USB enabled device. For example, the USB plug or socket may allow connection of the aerosol-generating device to a USB charging device to charge a rechargeable power supply within the aerosol-generating device. The USB plug or socket may additionally, or alternatively, support the transfer of data to or from, or both to and from, the aerosol-generating device. Additionally, or alternatively, the aerosol-generating device may be connected to a computer to transfer data to the device, such as new heating profiles for new aerosol-generating articles.

In those embodiments in which the aerosol-generating device comprises a USB plug or socket, the aerosol-generating device may further comprise a removable cover that covers the USB plug or socket when not in use. In embodiments in which the USB plug or socket is a USB plug, the USB plug may additionally or alternatively be selectively retractable within the device.

According to a third aspect of the present invention there is provided an aerosol-generating system comprising an aerosol-generating device according to the second aspect of the present invention, in accordance with any of the embodiments described herein. The aerosol-generating system also comprises an aerosol-generating article received within the device cavity.

The aerosol-generating article comprises an aerosol-forming substrate.

The aerosol-forming substrate may comprise a plug of tobacco. The tobacco plug may comprise one or more of: powder, granules, pellets, shreds, spaghettis, strips or sheets containing one or more of: tobacco leaf, fragments of tobacco ribs, reconstituted tobacco, homogenised tobacco, extruded tobacco and expanded tobacco. Optionally, the tobacco plug may contain additional tobacco or non-tobacco volatile flavour compounds, to be released upon heating of the tobacco plug. Optionally, the tobacco plug may also contain capsules that, for example, include the additional tobacco or non-tobacco volatile flavour compounds. Such capsules may melt during heating of the tobacco plug. Alternatively, or in addition, such capsules may be crushed prior to, during, or after heating of the tobacco plug.

The aerosol-generating article may have a total length of between approximately 30 millimetres and approximately 100 millimetres. The aerosol-generating article may have an external diameter of between approximately 5 millimetres and approximately 13 millimetres.

The aerosol-generating article may comprise a mouthpiece positioned downstream of the tobacco plug. The mouthpiece may be located at a downstream end of the aerosol-generating article. The mouthpiece may be a cellulose acetate filter plug. Preferably, the mouthpiece is approximately 7 millimetres in length, but can have a length of between approximately 5 millimetres to approximately 10 millimetres.

The tobacco plug may have a length of approximately 10 millimetres.

The diameter of the tobacco plug may be between approximately 5 millimetres and approximately 12 millimetres.

In a preferred embodiment, the aerosol-generating article has a total length of between approximately 40 millimetres and approximately 50 millimetres. Preferably, the aerosol-generating article has a total length of approximately 45 millimetres. Preferably, the aerosol-generating article has an external diameter of approximately 7.2 millimetres.

The invention will now be further described, by way of example only, with reference to the accompanying drawings in which:
Figure 1 shows a perspective view of a heater assembly according to an embodiment of the present invention;
Figure 2 shows a cross-sectional view of the heater assembly according to Figure 1 with the addition of a gasket; and
Figure 3 shows an aerosol-generating system including the heater assembly of Figure 1.

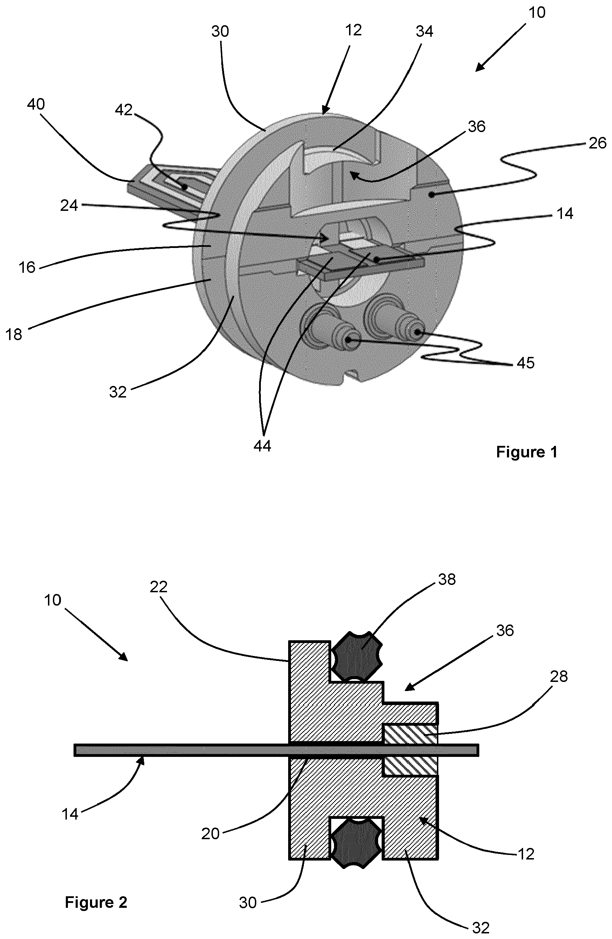

Figures 1 and 2 show a heater assembly 10 according to an embodiment of the present invention. The heater assembly 10 comprises a bushing 12 and an elongate electrical heater 14.

The bushing 12 comprises a first part 16 and a second part 18 secured together by an interference fit. The bushing 12 defines an aperture 20 at a first end 22 of the bushing 12 and a cavity 24 at a second end 26 of the bushing 12. A portion of the elongate electrical heater 14 extends through the aperture 20 and the cavity 24. The cavity 24 is filled with a potting material 28 to form a seal within the cavity 24 between the bushing 12 and the elongate electrical heater 14. The potting material 28 is omitted from Figure 1 to show the cavity 24.

The bushing 12 comprises a first flange 30 at the first end 22 of the bushing 12 and a second flange 32 at the second end 26 of the bushing 12. An annular groove 34 is formed between the first and second flanges 30, 32. A cut-out 36 is formed in the second flange 34.

The bushing 12 also comprises a gasket 38 in the form of an O-ring positioned within the annular groove 34. The gasket 38 is omitted from Figure 1 to shown the annular groove 34.

The elongate electrical heater 14 comprises an electrically insulating substrate 40 and a resistive heating element 42 formed on the electrically insulating substrate 40. The elongate electrical heater 14 also comprises two electrical contacts 44 for providing electrical connection between the resistive heating element 42 and control electronics of an aerosol-generating device comprising the heater assembly 10.

The heater assembly 10 comprises two pins 45 extending from the second end 26 of the bushing 12 to facilitate mechanical connection of the heater assembly 10 within an aerosol-generating device comprising the heater assembly 10.

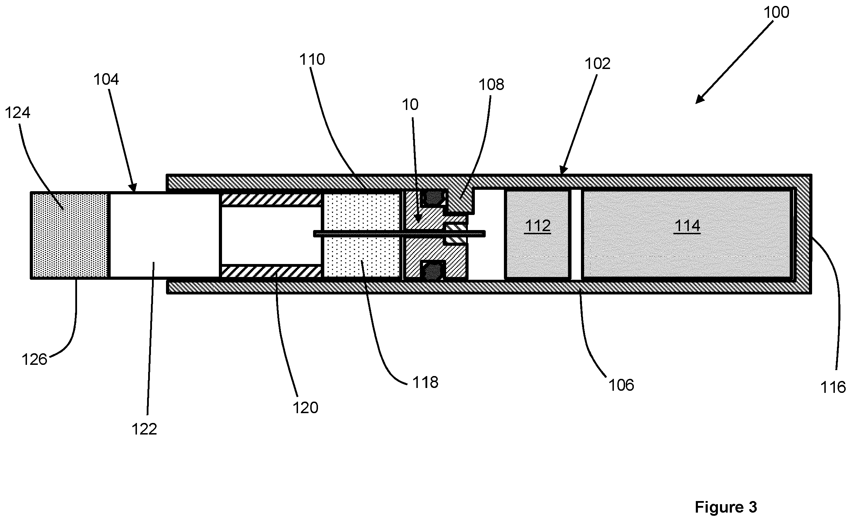

Figure 3 shows a cross-sectional view of an aerosol-generating system 100 comprising an aerosol-generating device 102 and an aerosol-generating article 104.

The aerosol-generating device 102 comprises a housing 106 and the heater assembly 10 positioned within the housing 106. The gasket 38 forms a seal between the bushing 12 and an inner surface of the housing 106. The housing 106 comprises a projection 108 that is received within the cut-out 36 of the bushing 12 to ensure correct orientation of the heater assembly 10 within the housing 106.

The housing 106 defines a device cavity 110 for receiving the aerosol-generating article 104, wherein the elongate electrical heater 14 extends from the first end 22 of the bushing 12 into the device cavity 110. A controller 112 and a power supply 114 are positioned within the housing 106 between the heater assembly 10 and a closed end 116 of the housing 106.

The aerosol-generating article 104 comprises an aerosol-forming substrate 118 in the form of a tobacco plug, a hollow acetate tube 120, a polymeric filter 122, a mouthpiece 124 and an outer wrapper 126. When an upstream end of the aerosol-generating article 104 is received within the device cavity 110 of the aerosol-generating device 102, the elongate electrical heater 14 of the heater assembly 10 is received within the aerosol-forming substrate 118.

During use of the aerosol-generating system 100, the controller 112 controls a supply of electrical power from the power supply 114 to the elongate electrical heater 14 to heat the aerosol-forming substrate 118 and form an aerosol for delivery to a user through the mouthpiece 124. The potting material 28 and the gasket 38 substantially prevent the leakage of liquid from the aerosol-forming substrate 118 into the portion of the housing 106 containing the controller 112 and the power supply 114.

## Claims

1. A heater assembly (10) for an aerosol-generating device (102), the heater assembly (10) comprising:
a bushing (12) defining an aperture (20) at a first end (22) of the bushing (12) and a cavity (24) at a second end (26) of the bushing (12), the bushing (12) comprising a first flange (30) extending around either the first end (22) of the bushing (12) or the second end (26) of the bushing (12); and
an elongate electrical heater (14) extending from the first end (22) of the bushing (12), wherein a portion of the elongate electrical heater (14) extends through the aperture (20) and into the cavity (24).

2. A heater assembly (10) according to claim 1, wherein the portion of the elongate electrical heater (14) extending through the aperture (20) is received within the aperture (20) by an interference fit.

3. A heater assembly (10) according to claim 1 or 2, wherein the first flange (30) extends around the first end (22) of the bushing (12), and wherein the bushing (12) further comprises a second flange (32) extending around the second end (26) of the bushing (12) and an annular groove (34) formed between the first flange (30) and the second flange (32).

4. A heater assembly (10) according to claim 3, wherein the heater assembly (10) comprises an annular gasket (38) positioned within the annular groove (34).

5. A heater assembly (10) according to any preceding claim, wherein the bushing (12) comprises a first end face through which the aperture (20) is formed, a second end face in which the cavity (24) is formed, and a cylindrical surface extending between the first end face and the second end face.

6. A heater assembly (10) according to claim 5 when dependent on claim 3 or 4, wherein the annular groove (34) is formed in the cylindrical surface.

7. A heater assembly (10) according to any preceding claim, wherein the heater assembly (10) comprises at least one electrical contact positioned at the second end (26) of the bushing (12), and wherein the at least one electrical contact is electrically connected to the elongate electrical heater (14).

8. A heater assembly (10) according to claim 7, wherein the at least one electrical contact comprises at least two electrical contacts.

9. A heater assembly (10) according to any preceding claim, wherein the cavity (24) is filled with a potting compound (28) to form a seal within the cavity (24) between the bushing (12) and the elongate electrical heater (14).

10. A heater assembly (10) according to claim 9, wherein the potting compound (28) comprises at least one of a thermoset polymer, an epoxy resin, silicone, and combinations thereof.

11. An aerosol-generating device (102) comprising:
a housing (106) defining a device cavity (110) for receiving an aerosol-generating article (104);
a heater assembly (10) according to any preceding claim, the heater assembly (10) positioned within the housing (106) at an upstream end of the device cavity (110), wherein the elongate electrical heater (14) extends from the first end (22) of the bushing (12) into the device cavity (110);
a power supply (114); and
a controller (112) for controlling a supply of electrical power from the power supply (114) to the elongate electrical heater (14).

12. An aerosol-generating device (102) according to claim 11, wherein the aerosol-generating device (102) comprises a gasket (38) positioned between the bushing (12) and the housing (106).

13. An aerosol-generating device (102) according to claim 12, wherein the gasket (38) is the annular gasket (38) according to claim 4.

14. An aerosol-generating system (100) comprising:
an aerosol-generating device (102) according to claim 11, 12 or 13; and
an aerosol-generating article (104) received within the device cavity (110).

## Patentansprüche

1. Heizvorrichtungsbaugruppe (10) für eine Aerosolerzeugungsvorrichtung (102), wobei die Heizvorrichtungsbaugruppe (10) Folgendes umfasst:
eine Buchse (12), die eine Öffnung (20) an einem ersten Ende (22) der Buchse (12) und einen Hohlraum (24) an einem zweiten Ende (26) der Buchse (12) definiert, wobei die Buchse (12) einen ersten Flansch (30) umfasst, der sich entweder um das erste Ende (22) der Buchse (12) oder das zweite Ende (26) der Buchse (12) erstreckt; und
eine längliche elektrische Heizvorrichtung (14), die sich von dem ersten Ende (22) der Buchse (12) erstreckt, wobei sich ein Abschnitt der länglichen elektrischen Heizvorrichtung (14) durch die Öffnung (20) und in den Hohlraum (24) erstreckt.

2. Heizvorrichtungsbaugruppe (10) nach Anspruch 1, wobei der Abschnitt der länglichen elektrischen Heizvorrichtung (14), der sich durch die Öffnung (20) erstreckt, durch eine Presspassung innerhalb der Öffnung (20) aufgenommen wird.

3. Heizvorrichtungsbaugruppe (10) nach Anspruch 1 oder 2, wobei sich der erste Flansch (30) um das erste Ende (22) der Buchse (12) erstreckt und wobei die Buchse (12) ferner einen zweiten Flansch (32), der sich um das zweite Ende (26) der Buchse (12) erstreckt, und eine ringförmige Nut (34), die zwischen dem ersten Flansch (30) und dem zweiten Flansch (32) gebildet ist, umfasst.

4. Heizvorrichtungsbaugruppe (10) nach Anspruch 3, wobei die Heizvorrichtungsbaugruppe (10) eine ringförmige Dichtung (38) umfasst, die innerhalb der ringförmigen Nut (34) positioniert ist.

5. Heizvorrichtungsbaugruppe (10) nach einem beliebigen vorhergehenden Anspruch, wobei die Buchse (12) eine erste Endfläche, durch die hindurch die Öffnung (20) gebildet ist, eine zweite Endfläche, in der der Hohlraum (24) gebildet ist, und eine zylindrische Oberfläche, die sich zwischen der ersten Endfläche und der zweiten Endfläche erstreckt, umfasst.

6. Heizvorrichtungsbaugruppe (10) nach Anspruch 5, wenn abhängig von Anspruch 3 oder 4, wobei die ringförmige Nut (34) in der zylindrischen Oberfläche gebildet ist.

7. Heizvorrichtungsbaugruppe (10) nach einem beliebigen vorhergehenden Anspruch, wobei die Heizvorrichtungsbaugruppe (10) wenigstens einen elektrischen Kontakt umfasst, der an dem zweiten Ende (26) der Buchse (12) positioniert ist, und wobei der wenigstens eine elektrische Kontakt elektrisch mit der länglichen elektrischen Heizvorrichtung (14) verbunden ist.

8. Heizvorrichtungsbaugruppe (10) nach Anspruch 7, wobei der wenigstens eine elektrische Kontakt wenigstens zwei elektrische Kontakte umfasst.

9. Heizvorrichtungsbaugruppe (10) nach einem beliebigen vorhergehenden Anspruch, wobei der Hohlraum (24) mit einer Vergussmasse (28) gefüllt ist, um eine Dichtung innerhalb des Hohlraums (24) zwischen der Buchse (12) und der länglichen elektrischen Heizvorrichtung (14) zu bilden.

10. Heizvorrichtungsbaugruppe (10) nach Anspruch 9, wobei die Vergussmasse (28) wenigstens eines von einem wärmehärtenden Polymer, einem Epoxidharz, Silikon und Kombinationen davon umfasst.

11. Aerosolerzeugungsvorrichtung (102), umfassend:
ein Gehäuse (106), das einen Vorrichtungshohlraum (110) zum Aufnehmen eines aerosolerzeugenden Artikels (104) definiert;
Heizvorrichtungsbaugruppe (10) nach einem beliebigen vorhergehenden Anspruch, wobei die Heizvorrichtungsbaugruppe (10) an einem vorgelagerten Ende des Vorrichtungshohlraums (110) innerhalb des Gehäuses (106) positioniert ist, wobei sich die längliche elektrische Heizvorrichtung (14) von dem ersten Ende (22) der Buchse (12) in den Vorrichtungshohlraum (110) hinein erstreckt;
eine Energieversorgung (114); und
eine Steuerung (112) zum Regeln einer Zufuhr von elektrischer Energie von der Energieversorgung (114) zu der länglichen elektrischen Heizvorrichtung (14).

12. Aerosolerzeugungsvorrichtung (102) nach Anspruch 11, wobei die Aerosolerzeugungsvorrichtung (102) eine Dichtung (38) umfasst, die zwischen der Buchse (12) und dem Gehäuse (106) positioniert ist.

13. Aerosolerzeugungsvorrichtung (102) nach Anspruch 12, wobei die Dichtung (38) die ringförmige Dichtung (38) nach Anspruch 4 ist.

14. Aerosolerzeugungssystem (100), umfassend:
eine Aerosolerzeugungsvorrichtung (102) nach Anspruch 11, 12 oder 13; und
einen aerosolerzeugenden Artikel (104), der innerhalb des Vorrichtungshohlraums (110) aufgenommen ist.

## Revendications

1. Ensemble de chauffage (10) pour un dispositif de génération d'aérosol (102), l'ensemble de chauffage (10) comprenant :
une douille (12) définissant une ouverture (20) au niveau d'une première extrémité (22) de la douille (12) et une cavité (24) au niveau d'une deuxième extrémité (26) de la douille (12), la douille (12) comprenant une première bride (30) s'étendant autour soit de la première extrémité (22) de la douille (12) soit de la deuxième extrémité (26) de la douille (12) ; et
un dispositif de chauffage électrique allongé (14) s'étendant depuis la première extrémité (22) de la douille (12), dans lequel une portion de dispositif de chauffage électrique allongé (14) s'étend à travers l'ouverture (20) et jusque dans la cavité (24).

2. Ensemble de chauffage (10) selon la revendication 1, dans lequel la portion du dispositif de chauffage électrique allongé (14) s'étendant à travers l'ouverture (20) est reçue au sein de l'ouverture (20) par un ajustement avec serrage.

3. Ensemble de chauffage (10) selon la revendication 1 ou 2, dans lequel la première bride (30) s'étend autour de la première extrémité (22) de la douille (12), et dans lequel la douille (12) comprend en outre une deuxième bride (32) s'étendant autour de la deuxième extrémité (26) de la douille (12) et une rainure annulaire (34) formée entre la première bride (30) et la deuxième bride (32).

4. Ensemble de chauffage (10) selon la revendication 3, dans lequel l'ensemble de chauffage (10) comprend un joint annulaire (38) positionné au sein de la rainure annulaire (34).

5. Ensemble de chauffage (10) selon une quelconque revendication précédente, dans lequel la douille (12) comprend une première face d'extrémité à travers laquelle l'ouverture (20) est formée, une deuxième face d'extrémité dans laquelle la cavité (24) est formée et une surface cylindrique s'étendant entre la première face d'extrémité et la deuxième face d'extrémité.

6. Ensemble de chauffage (10) selon la revendication 5 lorsqu'elle dépend de la revendication 3 ou 4, dans lequel la rainure annulaire (34) est formée dans la surface cylindrique.

7. Ensemble de chauffage (10) selon une quelconque revendication précédente, dans lequel l'ensemble de chauffage (10) comprend au moins un contact électrique positionné au niveau de la deuxième extrémité (26) de la douille (12), et dans lequel l'au moins un contact électrique est raccordé électriquement au dispositif de chauffage électrique allongé (14).

8. Ensemble de chauffage (10) selon la revendication 7, dans lequel l'au moins un contact électrique comprend au moins deux contacts électriques.

9. Ensemble de chauffage (10) selon une quelconque revendication précédente, dans lequel la cavité (24) est remplie d'un matériau d'enrobage (28) pour former un joint d'étanchéité au sein de la cavité (24) entre la douille (12) et le dispositif de chauffage électrique allongé (14).

10. Ensemble de chauffage (10) selon la revendication 9, dans lequel le matériau d'enrobage (28) comprend au moins l'un parmi un polymère thermodurci, une résine époxy, une silicone et des combinaisons de ceux-ci.

11. Dispositif de génération d'aérosol (102) comprenant :
un logement (106) définissant une cavité de dispositif (110) destinée à recevoir un article de génération d'aérosol (104) ;
un ensemble de chauffage (10) selon une quelconque revendication précédente, l'ensemble de chauffage (10) étant positionné au sein du logement (106) au niveau d'une extrémité amont de la cavité de dispositif (110), dans lequel le dispositif de chauffage électrique allongé (14) s'étend depuis la première extrémité (22) de la douille (12) jusque dans la cavité de dispositif (110) ;
une alimentation électrique (114) ; et
un dispositif de commande (112) destiné à commander une alimentation en puissance électrique depuis l'alimentation électrique (114) vers le dispositif de chauffage électrique allongé (14).

12. Dispositif de génération d'aérosol (102) selon la revendication 11, dans lequel le dispositif de génération d'aérosol (102) comprend un joint (38) positionné entre la douille (12) et le logement (106).

13. Dispositif de génération d'aérosol (102) selon la revendication 12, dans lequel le joint (38) est le joint annulaire (38) selon la revendication 4.

14. Système de génération d'aérosol (100) comprenant :
un dispositif de génération d'aérosol (102) selon la revendication 11, 12 ou 13 ; et
un article de génération d'aérosol (104) reçu au sein de la cavité de dispositif (110).
